# EUROPEAN PATENT APPLICATION

(11) **EP 3 184 446 A1**
(43) Date of publication of application: **28.06.2017**
(21) Application number: 15833379.9
(22) Date of filing: 10.08.2015
(51) Int. Cl.: B65B 55/16, A61B 50/20, A61F 2/82, A61M 25/10

(54) **METHOD FOR MANUFACTURING PACKAGED MEDICAL TOOL**

(30) Priority: 22.08.2014 JP 2014169424
(71) Applicant: Terumo Kabushiki Kaisha, Tokyo 151-0072 (JP)
(72) Inventor: KAWASHIMA, Yoshio, Fujinomiya-shi Shizuoka 418-0015 (JP)
(74) Representative: Casalonga
(86) International application number: PCT/JP2015/072614
(87) International publication number: WO 2016/027712

(57) **Abstract**

[Problem] A method for manufacturing a packaged medical device, which is capable of hermetically packaging a medical device in a low-oxygen atmosphere and also temporarily raising the oxygen concentration without requiring time management, is provided.

[Means for Resolution] This method for manufacturing a packaged medical device includes a sealing step of preparing a packaging bag 20 having a first sub-chamber 21 and a second sub-chamber 22 that are separated from each other, the first sub-chamber 21 being caused to contain a medical device 10 and a deoxygenating agent 25 and then being sealed, and the second sub-chamber 22 being sealed in a higher oxygen concentration than that in the first sub-chamber 21, a communication step of causing the first sub-chamber 21 and the second sub-chamber 22 to communicate with each other, and an irradiation step of irradiating the packaging bag 20 with radiation after the communication step.

## Description

### [Technical Field]

The present invention relates to a method for manufacturing a packaged medical device. In particular, the invention relates to a method for manufacturing a medical device that is desired to be located in a low-oxygen atmosphere.

### [Background Art]

A medical device, such as a catheter, may be made from a material that is affected by oxygen, such as a biological physiological active substance, for example, anticancer drug (hereinafter referred to as a "drug") and a biodegradable polymer. Examples of such devices include a drug eluting balloon and a drug eluting stent. It is desirable that such medical devices be located in a low-oxygen atmosphere so as to be prevented as much as possible from being exposed to oxygen at the time of transportation or storage. Therefore, the medical device is contained in a packaging bag made from a material that does not permeate oxygen, the inside of the packaging bag is brought into a low-oxygen state, for example, by filling with nitrogen, and then, the packaging bag is sealed.

Furthermore, medical devices are required to be sterilized after being manufactured, so as to prevent the infection with bacteria. Examples of the method of sterilizing a medical device include methods using heat sterilization, gaseous sterilization, and radiation sterilization including electron beam sterilization. Among these, radiation sterilization is able to be performed even in a state in which the medical device is packaged, thus enabling simplifying a sterilization process. For example, Patent Literature 1 discloses hermetically packaging an article including plastic together with a deoxygenating agent and performing electron beam sterilization. This sterilization method enables sterilizing the hermetically packaged article under a low-oxygen atmosphere.

### [Citation List]

### [Patent Literature]

[Patent Literature 1] JP-A-H08-89561

### [Summary of the Invention]

### [Problems that the Invention is to Solve]

A medical device, such as a catheter, may be made from a material that is affected by oxygen, such as a drug or a biodegradable polymer. Therefore, it is desirable that, as in the above-mentioned Patent Literature 1, the hermetically packaged article be sterilized under a low-oxygen atmosphere. On the other hand, in the case of radiation sterilization, in particular, electron beam sterilization, performing radiation sterilization in an oxygen atmosphere can enhance a sterilization effect. More specifically, in the case where radiation sterilization is performed in an oxygen atmosphere, sterilization can be performed with a small dose of radiation. Therefore, with regard to a method for sterilizing a medical device, the method of performing radiation sterilization under a low-oxygen atmosphere is higher in sterilization efficiency and more desirable than such a sterilization method as that disclosed in the above-mentioned Patent Literature 1. Therefore, with regard to a method for manufacturing a packaged medical device, satisfying both the attainment of a low-oxygen atmosphere in a packaging bag and the improvement in sterilization effect of a medical device was an unresolved problem.

To resolve this problem, for example, a method can be considered which includes sealing a packaging bag in which a medical device and a deoxygenating agent are contained in a low-oxygen atmosphere, performing irradiation with radiation immediately after sealing, and after that, lowering the oxygen concentration of the inside of the packaging bag by the deoxygenating agent, thus keeping the oxygen concentration in the packaging bag during irradiation with radiation and, after that, lowering the oxygen concentration in the packaging bag into a low-oxygen state. However, with such a method, unless time management during a period from sealing of the packaging bag to irradiation with radiation is strictly performed, the oxygen concentration in the packaging bag during irradiation with radiation is likely to become too low. Therefore, there are problems in that the burden of time management during a period from packaging to irradiation with radiation is large and the degree of freedom of a process from manufacturing to irradiation with radiation becomes low.

The invention has been made to solve the above-mentioned problems, and thus has an object to provide a method for manufacturing a packaged medical device, which is capable of hermetically packaging a medical device in a low-oxygen atmosphere and also temporarily raising the oxygen concentration without requiring time management.

### [Means for Solving the Problems]

A method for manufacturing a packaged medical device according to the invention, which attains the above-mentioned object, includes a sealing step of preparing a packaging bag having a first sub-chamber and a second sub-chamber that are separated from each other, the first sub-chamber being caused to contain a medical device and a deoxygenating agent and then being sealed, and the second sub-chamber being sealed in a higher oxygen concentration than that in the first sub-chamber, a communication step of causing the first sub-chamber and the second sub-chamber to communicate with each other, and an irradiation step of irradiating the packaging bag with radiation after the communication step.

### [Advantage of the Invention]

In the method for manufacturing a packaged medical device configured as described above, since, in the sealing step, the packaging bag having the first sub-chamber and the second sub-chamber that are separated from each other, the first sub-chamber being caused to contain the medical device and the deoxygenating agent and the second sub-chamber being set to a higher oxygen concentration than that in the first sub-chamber, is provided, and the communication step and the irradiation step are performed after the sealing step, the oxygen concentration in a region in which the medical device is contained can be temporarily raised at optional timing after the sealing step, and the medical device can be placed in a low-oxygen atmosphere at time points other than the optional timing. Accordingly, the timing of irradiation with radiation can be optionally set with the medical device contained in the sealed state, time management in a process from manufacturing of the medical device to irradiation with radiation can be made unnecessary, and the sterilization effect on the medical device by irradiation with radiation can be enhanced. Moreover, a time period for which the medical device is located under an oxygen atmosphere can be reduced to the minimum.

The medical device can be configured to have a drug loading portion in which a drug is loaded. The oxygen concentration in the sealed bag temporarily increases in the communication step, but decreases with time due to the deoxygenating agent after the communication step. Therefore, the medical device is located under a low-oxygen atmosphere in the sealing step and for a period from the irradiation step to opening of the packaging bag. Accordingly, the risk of the drug of the medical device decreasing in beneficial effect due to an influence of oxygen, such as oxidation reaction, can be reduced. In other words, in the method for manufacturing a medical device according to the invention, the risk of the beneficial effect of a drug decreasing before the medical device is used for a patient can be reduced.

When a partition portion is formed inside the packaging bag and the first sub-chamber and the second sub-chamber are separated from each other by the partition portion, two spaces different in oxygen concentration can be formed in a single packaging bag. Therefore, in the method for manufacturing a medical device according to the invention, the packaging bag, in which the first sub-chamber and the second sub-chamber are formed, is able to have a structure facilitating the communication step. Moreover, since breaking the partition portion in the packaging bag enables the first sub-chamber and the second sub-chamber to communicate with each other, the communication step can be facilitated.

When the partition portion is formed by opposite inner surfaces of the packaging bag being subjected to thermal fusion bonding, the partition portion can be easily formed.

When the partition portion is formed in such a manner that the fusion bonding strength thereof is lower than that of a peripheral portion of the packaging bag, pressing the packaging bag with a moderate force enables easily breaking only the partition portion, so that the communication step can be further facilitated.

When, in the communication step, at least a part of the partition portion is broken by pressing the second sub-chamber to cause the first sub-chamber and the second sub-chamber to communicate with each other, the first sub-chamber and the second sub-chamber can be caused to communicate with each other without pressing the first sub-chamber, in which the medical device is contained. Therefore, in the method for manufacturing a medical device according to the invention, the medical device can be prevented from being damaged when the packaging bag is pressed in the communication step.

When radiation used for irradiation in the irradiation step is an electron beam, the medical device can be effectively sterilized under an oxygen atmosphere. Moreover, in the case of electron beam sterilization, for example, heat is hardly generated by electron beam irradiation, and sterilization can be performed in a short time.

When the medical device is a catheter having a drug eluting stent or a drug eluting balloon, since a drug applied to the stent or the balloon is located under a low-oxygen atmosphere at other than the time of irradiation with radiation, a decrease in beneficial effect can be reduced. More specifically, in a case where a catheter having a drug eluting stent or a drug eluting balloon is hermetically packaged, the risk of a drug being deteriorated by, for example, oxidation reaction during storage due to oxygen in the packaging bag can be reduced.

### [Brief Description of the Drawings]

Fig. 1 is a plan view of a packaging bag in a state obtained before a partition portion is broken after a medical device is hermetically packaged.
Fig. 2 is a front view of the packaging bag in a state obtained before the partition portion is broken after the medical device is hermetically packaged.
Fig. 3 is a front view of the packaging bag in a state obtained after the partition portion is broken.
Fig. 4 is a plan view of a packaging bag at a stage in which a medical device and a deoxygenating agent are contained inside the packaging bag.
Fig. 5 is a plan view of the packaging bag at a stage in which a first sub-chamber is formed.
Fig. 6 is a plan view of the packaging bag at a stage in which a second sub-chamber is formed.
Fig. 7 is a front view illustrating a process proceeding from an increase in oxygen concentration in the packaging bag to sterilization.
Fig. 8 is a graph illustrating the transition of oxygen concentration with time in the packaging bag.
Fig. 9 is a plan view of a packaging bag according to a first modification example.
Fig. 10 is a front view of a packaging bag according to a second modification example.

### [Mode for Carrying Out the Invention]

Hereinafter, embodiments of the invention will be described with reference to the drawings. Moreover, dimensional ratios illustrated in the drawings are exaggerated for the purpose of illustration and may be different from the actual ratios. Furthermore, in the present specification, the side at which a medical device is inserted into the living body lumen is referred to as a "distal end" or a "distal side", and the near side at which a manipulation is performed is referred to as a "proximal end" or a "proximal side".

A method for manufacturing a packaged medical device according to an embodiment of the invention is a method of causing a balloon catheter 10, on which a stent 12 having a drug loading portion in which a drug is loaded is mounted, to be hermetically contained in a packaging bag 20 and then performing sterilization by irradiation with radiation on the balloon catheter 10.

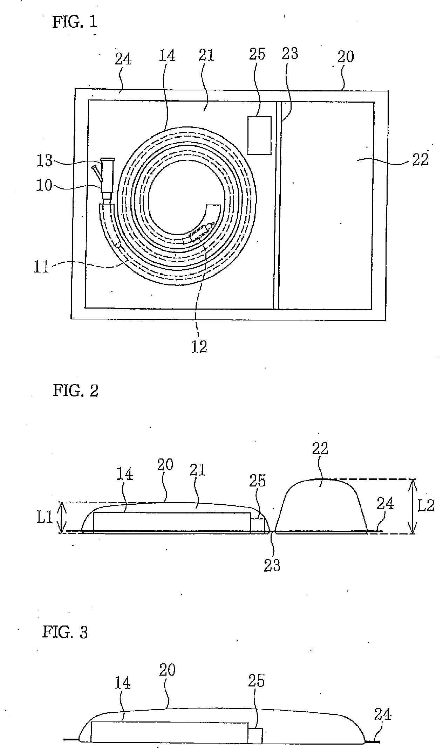

As illustrated in Fig. 1 and Fig. 2, the packaging bag 20, which is made from a material that does not permeate gas, such as oxygen, is hermetically sealed in a gas-impermeable state at a peripheral portion 24. The inside of the packaging bag 20 is partitioned with a partition portion 23 into a first sub-chamber 21 and a second sub-chamber 22, which are separated from each other in such a way as to prevent circulation of gas therebetween. The balloon catheter 10 and a deoxygenating agent 25 are contained in the first sub-chamber 21. The inside of the first sub-chamber 21 is filled with nitrogen, and is thus in a state in which the oxygen concentration is lower than that of air. In other words, the balloon catheter 10 is located in a low-oxygen atmosphere inside the packaging bag 20. The inside of the second sub-chamber 22 is filled with a gas the oxygen concentration of which is higher than that inside the first sub-chamber 21.

The packaging bag 20 is hermetically sealed by two film-like sheets being fused and bonded at the peripheral portion 24. Moreover, the partition portion 23, which is formed between the first sub-chamber 21 and the second sub-chamber 22, is also formed by opposite inner surfaces of the packaging bag 20 being fused and bonded. Furthermore, the first sub-chamber 21 is formed in such a way as to have a thickness L1 required to contain the balloon catheter 10 and the deoxygenating agent 25 between two sheets, as illustrated in Fig. 2, in the hermetically-sealed packaging bag 20. Moreover, the second sub-chamber 22 is formed in such a way as to have a thickness L2, which is greater than that of the first sub-chamber 21, to increase the volume of the second sub-chamber 22 between two sheets, as illustrated in Fig. 2, in the hermetically-sealed packaging bag 26.

The partition portion 23 is formed with a width narrower than that of the peripheral portion 24 in such a manner that the fusion bonding strength thereof is lower than that of the peripheral portion 24 of the packaging bag 20, and is able to be broken by pressing of the second sub-chamber 22 at an optional time point after a sealing step for the packaging bag 20. As illustrated in Fig. 3, when the partition portion 23 is broken, the first sub-chamber 21 and the second sub-chamber 22 are caused to communicate with each other, so that the entire packaging bag 20 forms a single chamber.

As illustrated in Fig. 1, the balloon catheter 10, which is contained in the packaging bag 20, includes a long and hollow catheter main body portion 11, a balloon provided in proximity to the distal end of the catheter main body portion 11, a stent 12 mounted on an outer circumferential surface of the balloon, and a hub 13 fixed to the proximal end of the catheter main body portion 11. Furthermore, the balloon catheter illustrated in Fig. 1 has an Over-the-Wire structure, but is not limited to this structure and can be, for example, a balloon catheter having a Rapid Exchange structure.

A holder tube 14 is provided to protect the balloon catheter 10, which is contained inside the holder tube 14, from, for example, contact with or impact from the outside, and is formed in such a way as to cover the balloon catheter 10. Moreover, the holder tube 14 is contained in the packaging bag 20 in a spiral shape.

As illustrated in Fig. 1, the balloon catheter 10 includes the hub 13, the catheter main body portion 11, the balloon, and the stent 12.

The catheter main body portion 11 is composed of an outer tube, which is a tube-shaped body the distal end and the proximal end of which are opened, and an inner tube, which is located inside the outer tube. An inflation lumen through which a fluid for inflating the balloon flows is formed inside the outer tube, and a guide wire lumen through which a guide wire is inserted is formed in the inner tube. The material used to make the inner tube or the outer tube is desirably a material having a certain degree of flexibility, and examples of such a material to be used include polyolefin, such as polyethylene, polypropylene, polybutene, ethylenepropylene copolymer, ethylene-vinyl acetate copolymer, ionomer, or a mixture of two or more of these materials, thermoplastic resin, such as soft polyvinyl chloride resin, polyamide, polyamide elastomer, polyester, polyester elastomer, polyurethane, and fluorine resin, silicone rubber, and latex rubber.

The hub 13 includes a first opening portion, to which an indeflator (a pressure applying device) that communicates with the inflation lumen of the outer tube to supply a fluid for inflating the balloon is connected, and a second opening portion, which communicates with the guide wire lumen of the inner tube. The fluid for inflating the balloon is, for example, contrast agent, physiological salt solution, or a mixture of these fluids. Examples of the material used to make the hub include thermoplastic resin, such as polycarbonate, polyamide, polysulfone, polyarylate, and methacrylate-butylene-styrene copolymer.

The balloon is mounted in a folded state (or in a deflated state), and is inflated by the above-mentioned fluid being introduced. The inside of the balloon communicates with the inflation lumen of the outer tube. Accordingly, the fluid, which has flowed in from the first opening portion of the hub, flows in the inside of the balloon, so that the balloon is inflated. The material used to make the balloon is desirably a material having flexibility, and examples of such a material include a polymer material, such as polyolefin, a crosslinked polymer of polyolefin, polyester, polyester elastomer, polyvinyl chloride, polyurethane, polyurethane elastomer, polyphenylenesulfide, polyamide, polyamide elastomer, and fluorine resin, silicone rubber, and latex rubber. Polyester is, for example, polyethylene terephthalate. The material used to make the balloon is not limited to an embodiment in which the above-mentioned polymer material is solely used, but can be, for example, a film obtained by stacking the above-mentioned polymer materials in layers as appropriate.

The stent 12, when placed in close contact with the inner surface of a stenosed portion, which is a lesion, keeps the living body lumen in an appropriate size. Moreover, the stent 12 expands according to the inflation of the balloon. Furthermore, the stent 12 has a drug loading portion, which has a drug, formed on the outside surface of the stent 12.

The stent 12 is made from a material having biocompatibility. Examples of the material having biocompatibility include iron, titanium, aluminum, tin, tantalum or a tantalum alloy, platinum or a platinum alloy, gold or a gold alloy, a titanium alloy, a nickel-titanium alloy, a cobalt-base alloy, a cobalt-chrome alloy, stainless steel, a zinc-tungsten alloy, and a niobium alloy. Moreover, the stent can be made from a biodegradable polymer. For example, the biodegradable polymer can be composed of poly(L-lactide), poly(D-lactide), polyglycolide, or poly(L-lactide-co-glycoside).

The drug, which is coated on the outside surface of the stent 12, is used to prevent restenosis or reocclusion of the living body lumen, which may occur during placement of the stent 12 at the lesion. In the present embodiment, a mixture of the drug and the biodegradable polymer is used to form the drug loading portion. Furthermore, the drug loading portion is not limited to being provided on the outside surface of the stent, but can be provided on the inside surface of the stent or on both the outside surface and the inside surface.

The drug is desirably at least one drug selected from the group consisting of, for example, anticancer drug, immunosuppressive drug, antibiotic, antirheumatic, antithrombogenic drug, HMG-CoA reductase inhibitor, angiotensin-converting enzyme inhibitor, calcium antagonist agent, antihyperlipidemic drug, integrin inhibitor, anti-allergic drug, antioxidant agent, GpIIb/IIIa inhibitor, retinoid, flavonoid, carotenoid, lipid improving drug, DNA synthesis inhibitor, tyrosine kinase inhibitor, antiplatelet drug, anti-inflammatory drug, a tissue-derived biomaterial, interferon, and NO production promoting substance, for the reason that the drug is able to control the behavior of cells of a lesional tissue to treat the lesion. In particular, when the drug is immunosuppressive drug and it is rapamycin or a derivative thereof, the effect of preventing restenosis or reocclusion of the living body lumen becomes higher.

The biodegradable polymer, which is mixed with the drug, only needs to be the one that gradually biologically degrades after the stent 12 is placed at the lesion and has no adverse influence on the living body. Examples of such a biodegradable polymer include at least one polymer selected from the group consisting of polyglycolic acid, polylactic acid, polycaprolactone, polyhydroxybutyrate, cellulose, polyhydroxybutyrate valeric acid, and polyorthoester, and a copolymer, a mixture, and a compound of some of these polymers.

The composition ratio (mass ratio) between the drug and the biodegradable polymer can be set to 1:99 to 99:1, but, when it is set to 30:70 to 70:30, as much drug as possible can be loaded while the physical property and separation performance of the biodegradable polymer are taken into consideration.

Such a mixture of the drug and the biodegradable polymer can be attached to the surface of the stent main body in the following method. First, the drug and the biodegradable polymer with the composition ratio such as that described above are dissolved in a solvent, such as acetone, ethanol, chloroform, or tetrahydrofuran in such a manner that the solution concentration becomes 0.001% to 20% by mass or, desirably, 0.01% to 10% by mass. This solution is applied to the surface of the stent main body using, for example, a spray or a dispenser and the solvent is volatized, so that a layer of the mixture of the drug and the biodegradable polymer is formed on the surface of the stent. However, this method is not limiting, but an optional method can be employed.

The drug is able to be present on the surface of the stent main body in an optional embodiment, but, when a layer is formed on the surface of the stent as a mixture with the biodegradable polymer, the drug becomes able to be gradually released in the living body.

The deoxygenating agent 25, which is contained in the packaging bag 20, only needs to be the one that is conventionally enclosed and used in a package for the purpose of improving the preserving property of a medicinal product or preventing the deactivation or deterioration of a medical product. Examples of such a known deoxygenating agent include an iron-based one and an organic-based oxygen absorber, such as ascorbic acid. While such an oxygen absorber includes a type that absorbs only oxygen, a type that absorbs oxygen and carbon dioxide gas, and a type that absorbs oxygen and generates carbon dioxide gas, any type can be employed.

The deoxygenating agent 25 can be used while retaining an ordinarily-available powder state, but is desirably used while being packed in, for example, a bag having high permeability to gas. Moreover, it can be adjusted into a solid-agent form, such as a pill form, or a film-like or sheet-like form.

The size of the deoxygenating agent 25 in any form only needs to be of such a degree as to enable the deoxygenating agent 25 to be packed together with the balloon catheter 10 in the first sub-chamber 21 of the packaging bag 20. The number of deoxygenating agents 25 can also be optionally selected as appropriate, but is set to one in the present embodiment.

Furthermore, although not illustrated, a desiccant agent can be set in the first sub-chamber 21. This enables decreasing the possibility of the drug causing a hydrolysis reaction in a case where the medical device has a drug loading portion.

The packaging bag 20 is required to be made using a low gas-permeable substance having low permeability to oxygen. The packaging bag 20 is formed from a multi-layered film obtained by stacking a plurality of layers including a low gas-permeable substance. This enables attaining the strength required for the packaging bag 20 and lowering the permeability to oxygen.

A sheet used as a material of the packaging bag 20 in the present embodiment has a multi-layered structure in which a surface layer made from, for example, PET resin or nylon is formed on the outer surface side of an aluminum film and an inner surface layer made from, for example, polyethylene is formed on the inner surface side of the aluminum film.

Furthermore, for the purpose of improving the strength, an additional layer made from, for example, PET resin or nylon can be formed on the inner surface side of the aluminum film and an inner surface layer made from, for example, polyethylene can be formed on the inner surface side of the additional layer. In this way, the types or numbers of layers can be changed according to the strength required for the packaging bag 20. In that case, to reliably prevent damage to the aluminum film, it is desirable that the aluminum film be used as an intermediate layer of the sheet used as a material of the packaging bag 20.

The inner surface layer in the sheet used as a material of the packaging bag 20 is made from polyethylene, which has thermoplasticity as mentioned above, and is, therefore, melted by being heated at about 150° C to 160° C. At the time of forming the packaging bag 20, peripheral portions of two stacked sheets are heated to melt the inner surface layers, which are then fused and bonded to each other, so that the peripheral portion 24 is formed. Moreover, the partition portion 23 can also be formed by the inner surface layers being fused and bonded to each other in a similar way.

Next, a method of packaging the balloon catheter 10 (a medical device) is described.

First, a sealing step of preparing the packaging bag 20, which is sealed after the first sub-chamber 21 and the second sub-chamber 22 are formed therein, is performed. The sealing step includes a stage of causing the balloon catheter 10 to be contained in the packaging bag 20, a stage of forming the first sub-chamber 21, a stage of forming the second sub-chamber 22, a stage of sealing the packaging bag 20, and a stage of preparing the sealed packaging bag 20. Furthermore, the sealing step can include purchasing a packaging bag 20 for which the stage of causing the balloon catheter 10 to be contained in the packaging bag 20, the stage of forming the first sub-chamber 21, the stage of forming the second sub-chamber 22, and the stage of sealing the packaging bag 20 are completed, and then the subsequent steps can be performed.

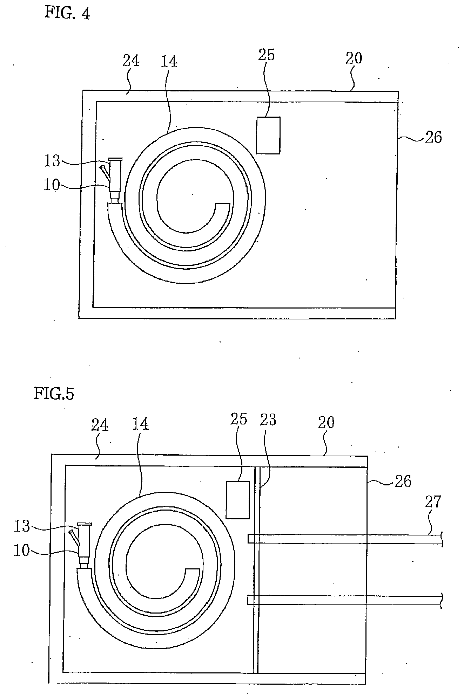

In the stage of causing the balloon catheter 10 to be contained in the packaging bag 20, as illustrated in Fig. 4, a packaging bag 20 in which a peripheral portion 24 is previously subjected to thermal fusion bonding at three sides thereof and only one side thereof used as the second sub-chamber 22 is set as an opening 26 is prepared. Then, the balloon catheter 10 and the deoxygenating agent 25 are inserted into the packaging bag 20 via the opening 26.

After the balloon catheter 10 and the deoxygenating agent 25 are contained in the packaging bag 20, as illustrated in Fig. 5, the partition portion 23 is formed by thermal fusion bonding, and at the same time, the first sub-chamber 21, which is formed by the partition portion 23 and the peripheral portion 24, is filled with nitrogen by the use of a nitrogen injection nozzle 27. With this, the balloon catheter 10 and the deoxygenating agent 25 are hermetically contained in the first sub-chamber 21, which is in a nitrogen atmosphere (the stage of forming the first sub-chamber 21).

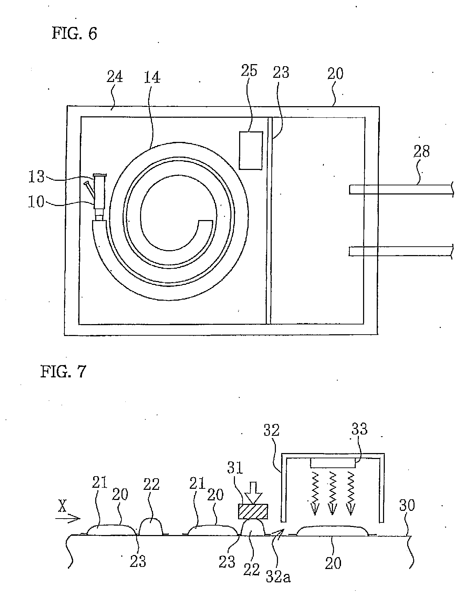

After the first sub-chamber 21 is formed, as illustrated in Fig. 6, the peripheral portion 24 is formed at the opening 26 by thermal fusion bonding, and at the same time, the second sub-chamber 22, which is formed by the partition portion 23 and the peripheral portion 24, is filled with gas including oxygen by the use of an oxygen injection nozzle 28. With this, the sealed second sub-chamber 22 enters a state in which the oxygen concentration is higher than that in the first sub-chamber 21 (the stage of forming the second sub-chamber 22 and the stage of sealing the packaging bag 20).

Here, the size and oxygen concentration of the second sub-chamber 22 relative to the packaging bag 20 can be set as appropriate according to the oxygen concentration needed when the entire packaging bag 20 has formed a single chamber due to the partition portion 23 being broken. For example, if the volume of the second sub-chamber 22 is large, the second sub-chamber 22 can be filled with air. Conversely, if the second sub-chamber 22 is filled with pure oxygen, the required volume of the second sub-chamber 22 can be set small. Furthermore, while, in the present embodiment, the second sub-chamber 22 is formed larger in thickness than the first sub-chamber 21, as long as the volume required for the second sub-chamber 22 is able to be secured, the thickness of the second sub-chamber 22 can be equal to or smaller than that of the first sub-chamber 21.

The procedure of the sealing step is not limited to this example. For example, the procedure can include previously forming an opening 26 at the side used as the first sub-chamber 21, first filling the second sub-chamber 22 with gas including oxygen and, at the same time, forming the partition portion 23, then inserting the balloon catheter 10 and the deoxygenating agent 25 via the opening 26, and filling the first sub-chamber 21 with nitrogen and, at the same time, forming the peripheral portion 24 at the opening 26 to seal the first sub-chamber 21. In any case, a packaging bag 20 only needs to be able to be prepared which has the first sub-chamber 21 and the second sub-chamber 22 that are separated from each other, the first sub-chamber 21 being caused to contain the balloon catheter 10 and the deoxygenating agent 25 and then being sealed and the second sub-chamber 22 being sealed in a higher oxygen concentration than that in the first sub-chamber 21.

The sealed packaging bag 20 having the first sub-chamber 21 and the second sub-chamber 22 is prepared by the above-described method (the stage of preparing the sealed packaging bag 20), and then, the communication step and the irradiation step are performed. As illustrated in Fig. 7, the sealed packaging bag 20, in which the balloon catheter 10 is hermetically contained, is loaded on a conveyor belt 30 and is then conveyed in the direction of X illustrated in the figure. The conveyor belt 30 is provided with pressing means 31. As the pressing means 31, for example, a pressing device can be employed.

In the communication step, the pressing means 31 presses the second sub-chamber 22 of the packaging bag 20, which has been conveyed there. The pressure in the second sub-chamber 22 is raised by the pressing, and force is applied to thermal-fusing-bonded portions at four sides used to seal the second sub-chamber 22. Since, among the thermal-fusing-bonded portions at four sides defining the second sub-chamber 22, the partition portion 23 is narrower in with and lower in fusion bonding strength than the peripheral portion 24, only the partition portion 23 is broken by the pressing force of the pressing means 31. With this, the first sub-chamber 21 and the second sub-chamber 22 are caused to communicate with each other, gases in the respective sub-chambers are mixed, and the oxygen concentration in a sealed region containing the balloon catheter 10 becomes higher than before that time.

In the present embodiment, the second sub-chamber 22 is formed larger in the thickness L2 formed between two sheets than the first sub-chamber 21. Therefore, the pressing means 31 is able to easily press only the second sub-chamber 22 in a selective manner. With this, since the first sub-chamber 21, in which the balloon catheter 10 is contained, cannot be pressed, for example, the balloon catheter 10 can be prevented from being damaged by the pressing means 31.

The irradiation step is performed immediately after the communication step. An irradiation region 32 for irradiation with radiation is provided at the downstream side of the pressing means 31 on the conveyor belt 30. The irradiation region 32 has irradiation means 33, and the packaging bag 20, which is then located in the irradiation region 32, is irradiated with radiation emitted from the irradiation means 33. Moreover, the radiation used for irradiation in the irradiation step includes, for example, an electron beam, a gamma ray, and an X-ray. Among these, an electron beam is desirable from the viewpoint that sterilization can be performed in a short time.

The packaging bag 20, in which the first sub-chamber 21 and the second sub-chamber 22 have been caused to communicate with each other by the pressing means 31, becomes a single chamber in its entirety, and, therefore, becomes smaller in thickness than the second sub-chamber 22. Therefore, before the irradiation step is performed, it is possible to detect whether the partition portion 23 has been broken by the pressing means 31, by discriminating the largeness or smallness in thickness of the packaging bag 20.

Any means for discriminating the thickness of the packaging bag 20 can be employed. For example, the following means can be used. An entrance 32a of the irradiation region 32 is formed larger than the thickness of the packaging bag 20 obtained after the partition portion 23 is broken and smaller than the thickness of the second sub-chamber 22 obtained before the partition portion 23 is broken. Therefore, the packaging bag 20 that is able to pass through the entrance 32a of the irradiation region 32 can be regarded as the one in which the first sub-chamber 21 and the second sub-chamber 22 have been caused to communicate with each other by the partition portion 23 being broken, and thus can be advanced to the irradiation step. Conversely, the packaging bag 20 that is unable to pass through the entrance 32a of the irradiation region 32 may be the one in which the partition portion 23 is not broken and the first sub-chamber 21 and the second sub-chamber 22 do not communicate with each other, and thus can be excluded without being advanced to the irradiation region 32.

The irradiation time of electron beams in the irradiation step needs to be set in such a way as to secure the radiation dose required for sterilization of the balloon catheter 10, and is usually set to a value ranging from several seconds to one minute. The radiation dose for irradiation varies with the material or amount of an object to be irradiated, but is usually selected from a range of 2 kGy to 75 kGy.

Since the oxygen concentration in the region in which the balloon catheter 10 is hermetically contained is raised by the communication step, a sufficient sterilization effect by irradiation with electron beams can be obtained. It is considered that this is because oxygen being ozonized or radicalized by irradiation with electron beams enables supplementarily increasing the sterilization effect of electron beams themselves.

The packaging bag 20 in which the communication step and the irradiation step have been completed is in a state in which the balloon catheter 10 and the deoxygenating agent 25 are hermetically contained in a single chamber, and, after that, the oxygen concentration in the packaging bag 20 gradually decreases due to the deoxygenating agent 25. Eventually, the packaging bag 20 enters a state in which the balloon catheter 10 is hermetically contained in a low-oxygen atmosphere.

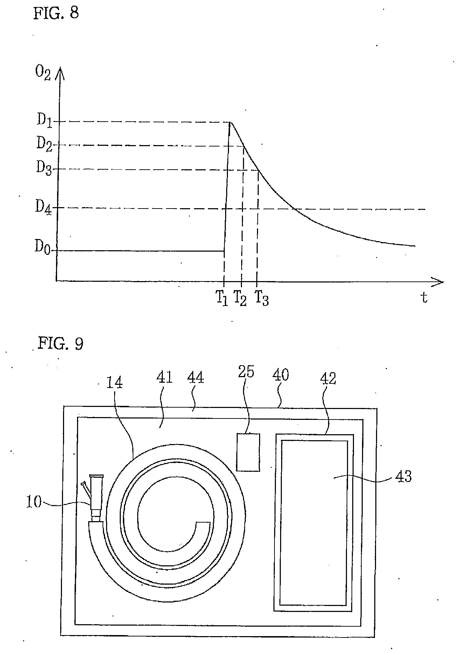

A description is made about how the oxygen concentration in the region in which the balloon catheter 10 is hermetically contained transitions in the communication step and the irradiation step and before and after those steps. In Fig. 8, the abscissa axis indicates time, and the ordinate axis indicates the oxygen concentration in the region in which the balloon catheter 10 is hermetically contained. The minimum oxygen concentration required at the time of irradiation with electron beams is denoted by D₄, the oxygen concentration in the first sub-chamber 21 of the packaging bag 20 is denoted by D₀, and D₀ is set smaller than D₄.

Time T₁ is the time at which the communication step has been performed, in other words, the time at which the partition portion 23 has been broken. At this time, the first sub-chamber 21 and the second sub-chamber 22 are caused to communicate with each other, so that the oxygen concentration in the region in which the balloon catheter 10 is hermetically contained rapidly increases and becomes D₁. After that, the oxygen concentration in the region in which the balloon catheter 10 is hermetically contained gradually decreases due to the enclosed deoxygenating agent 25.

The irradiation step is performed in a period from time T₂ to time T₃. The oxygen concentration at time T₂ is D₂ and the oxygen concentration at time T₃ is D₃, each of which is smaller than D₁ at the communication step, but is larger than the minimum oxygen concentration D₄ required at the time of irradiation with electron beams. Accordingly, in the irradiation step, sterilization of the balloon catheter 10 can be efficiently performed. According to the invention, since the first sub-chamber 21 and the second sub-chamber 22 that are separated from each other are formed in the packaging bag 20 and are configured to be able to communicate with each other at an optional time point, the communication step can be performed immediately before the irradiation step, and irradiation with electron beams can be performed in a state in which the oxygen concentration is sufficiently high.

On the other hand, since the oxygen concentration in the packaging bag 20 gradually decreases due to the deoxygenating agent 25 even after the irradiation step, the balloon catheter 10 can be eventually brought into a state of being hermetically contained in a low-oxygen atmosphere. Accordingly, a time period for which the drug of the stent 12 is affected by oxygen can be reduced to the minimum.

Next, a description is made about a first modification example of a packaging bag used in the method for manufacturing a medical device according to the invention. As illustrated in Fig. 9, a packaging bag 40 of the present modification example is formed as a first sub-chamber 41 in its entirety with a peripheral portion 44 at four sides, and the balloon catheter 10 and the deoxygenating agent 25 are contained in the first sub-chamber 41. Furthermore, an inner bag 42 having a second sub-chamber 43 is contained in the first sub-chamber 41. In this way, forming the first sub-chamber 41 and the second sub-chamber 43 with different bags enables bringing the first sub-chamber 41 and the second sub-chamber 43 into a state of being separated from each other without use of a partition portion. In the communication step, the inner bag 42 is broken by means, such as pressing, so that the first sub-chamber 21 and the second sub-chamber 22 can be caused to communicate with each other.

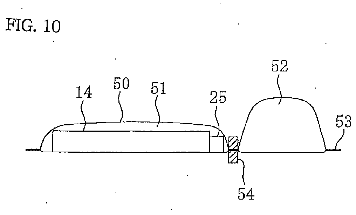

Next, a description is made about a second modification example of a packaging bag used in the method for manufacturing a medical device according to the invention. As illustrated in Fig. 10, a packaging bag 50 of the present modification example is formed to become a single region in its entirety with a peripheral portion 53 at four sides, and a sandwich portion 54 is provided at a middle portion of the packaging bag 50. The sandwich portion 54 is formed, for example, in such a clip-like shape as to be able to pinch and clamp the packaging bag 50 from the outside, and is thus configured to form a first sub-chamber 51 and a second sub-chamber 52 at both sides of the sandwich portion 54. The balloon catheter 10 and the deoxygenating agent 25 are contained at the side of the first sub-chamber 51. In the communication step, only removing the sandwich portion 54 enables causing the first sub-chamber 51 and the second sub-chamber 22 to communicate with each other.

As described above, the method for manufacturing a packaged medical device according to the present embodiment includes a sealing step of preparing a packaging bag 20 having a first sub-chamber 21 and a second sub-chamber 22 that are separated from each other, the first sub-chamber 21 being caused to contain a medical device and a deoxygenating agent 25 and then being sealed, and the second sub-chamber 22 being sealed in a higher oxygen concentration than that in the first sub-chamber 21, a communication step of causing the first sub-chamber 21 and the second sub-chamber 22 to communicate with each other, and an irradiation step of irradiating the packaging bag 20 with radiation after the communication step, so that, since, in the sealing step, the packaging bag 20 having the first sub-chamber 21 and the second sub-chamber 22 that are separated from each other, the first sub-chamber 21 being caused to contain the medical device and the deoxygenating agent 25 and the second sub-chamber 22 being set to a higher oxygen concentration than that in the first sub-chamber 21, is provided, and the communication step and the irradiation step are performed after the sealing step, the oxygen concentration in a region in which the medical device is contained can be temporarily raised at optional timing after the sealing step, and the medical device can be placed in a low-oxygen atmosphere at time points other than the optional timing. Accordingly, the timing of irradiation with radiation can be optionally set with the medical device contained in the sealed state, time management in a process from manufacturing of the medical device to irradiation with radiation can be made unnecessary, and a time period for which the medical device is located under an oxygen atmosphere can be reduced to the minimum.

Furthermore, the medical device can have a drug loading portion in which a drug is loaded. Here, the oxygen concentration in the sealed bag 20 temporarily increases in the communication step, but decreases with time due to the deoxygenating agent 25 after the communication step. Therefore, the medical device is located under a low-oxygen atmosphere in the sealing step and for a period from the irradiation step to opening of the packaging bag 20. Accordingly, the risk of the drug of the medical device decreasing in beneficial effect due to an influence of oxygen, such as oxidation reaction, can be reduced. In other words, in the method for manufacturing a packaged medical device according to the present embodiment, the risk of the beneficial effect of a drug decreasing before the medical device is used for a patient can be reduced.

Furthermore, when a partition portion 23 is formed inside the packaging bag 20 and the first sub-chamber 21 and the second sub-chamber 22 are separated from each other by the partition portion 23, two spaces different in oxygen concentration can be formed in a single packaging bag 20. Therefore, in the method for manufacturing a packaged medical device according to the present embodiment, the packaging bag 20, in which the first sub-chamber 21 and the second sub-chamber 22 are formed, is able to have a structure facilitating the communication step. Moreover, since breaking the partition portion 23 in the packaging bag 20 enables the first sub-chamber 21 and the second sub-chamber 22 to communicate with each other, the communication step can be facilitated.

Furthermore, when the partition portion 23 is formed by opposite inner surfaces of the packaging bag 20 being subjected to thermal fusion bonding, the partition portion 23 can be easily formed.

Furthermore, when the partition portion 23 is formed in such a manner that the fusion bonding strength thereof is lower than that of a peripheral portion 24 of the packaging bag 20, pressing the packaging bag 20 with a moderate force enables easily breaking only the partition portion 23, so that the communication step can be further facilitated.

Furthermore, when, in the communication step, at least a part of the partition portion 23 is broken by pressing the second sub-chamber 22 to cause the first sub-chamber 21 and the second sub-chamber 22 to communicate with each other, the first sub-chamber 21 and the second sub-chamber 22 can be caused to communicate with each other without pressing the first sub-chamber 21, in which the balloon catheter 10 is contained. Therefore, in the method for manufacturing a packaged medical device according to the present embodiment, the medical device can be prevented from being damaged when the packaging bag 20 is pressed in the communication step.

Furthermore, when radiation used for irradiation in the irradiation step is an electron beam, the balloon catheter 10 can be effectively sterilized under an oxygen atmosphere. Moreover, in the case of electron beam sterilization, for example, heat is hardly generated by electron beam irradiation, and sterilization can be performed in a short time.

Furthermore, when the balloon catheter 10 is a catheter having a drug eluting stent or a drug eluting balloon, since a drug applied to the stent or the balloon is located under a low-oxygen atmosphere at other than the time of irradiation with radiation, a decrease in beneficial effect can be reduced. More specifically, in a case where a catheter having a drug eluting stent or a drug eluting balloon is hermetically packaged, the risk of a drug being deteriorated by, for example, oxidation reaction during storage due to oxygen in the packaging bag 20 can be reduced.

Moreover, the invention is not limited to only the above-described embodiment, but can be changed in various manners by a person skilled in the art within a technical idea of the invention. For example, the medical device, which is contained in the packaging bag 20, is the balloon catheter 10 in the present embodiment, but can be a medical device other than that. Furthermore, the balloon catheter 10 in the present embodiment has a drug eluting stent 12, but can have a drug eluting balloon.

Furthermore, while, in the present embodiment, the fusion bonding strength of the partition portion 23 is set low and, when the second sub-chamber 22 is pressed in the communication step, only the partition portion 23 is broken without the peripheral portion 24 being broken, the partition portion 23 can be configured to be broken by other means. For example, the peripheral portion 24 of the packaging bag 20 can be previously sandwiched at four sides thereof by some means, and the second sub-chamber 22 can be pressed in that state to break the partition portion 23. In this case, the strength of the partition portion 23 does not necessarily need to be lower than that of the peripheral portion 24.

Furthermore, while, in the present embodiment, thermal fusion bonding is used as a method for forming the partition portion 23, opposite inner surfaces of the packaging bag 20 can be bonded by an adhesive material.

Furthermore, means for causing the first sub-chamber 51 and the second sub-chamber 22 to communicate with each other in the communication step is also not limited to the pressing means in the present embodiment. For example, suctioning both sides of the first sub-chamber 51 and the second sub-chamber 22 across the partition portion 23 can be used to break the partition portion 23.

This application is based on Japanese Patent Application No. 2014-169424 filed on August 22, 2014, the disclosed contents of which are hereby incorporated by reference herein in its entirety.

### [Description of Reference Numerals and Signs]

- 10: catheter,
- 11: catheter main body,
- 12: stent,
- 13: hub,
- 14: holder tube,
- 20: packaging bag,
- 21: first sub-chamber,

- 22: second sub-chamber,
- 23: partition portion,
- 24: peripheral portion,
- 25: deoxygenating agent,
- 26: opening,
- 30: conveyor belt,
- 31: pressing means,
- 32: irradiation region,
- 33: irradiation means.

## Claims

1. A method for manufacturing a packaged medical device, the method comprising:
a sealing step of preparing a packaging bag having a first sub-chamber and a second sub-chamber that are separated from each other, the first sub-chamber being caused to contain a medical device and a deoxygenating agent and then being sealed, and the second sub-chamber being sealed in a higher oxygen concentration than that in the first sub-chamber;
a communication step of causing the first sub-chamber and the second sub-chamber to communicate with each other; and
an irradiation step of irradiating the packaging bag with radiation after the communication step.

2. The method for manufacturing a packaged medical device according to claim 1, wherein the medical device has a drug loading portion in which a drug is loaded.

3. The method for manufacturing a packaged medical device according to claim 1 or 2, wherein a partition portion is formed in the packaging bag, and the first sub-chamber and the second sub-chamber are separated from each other by the partition portion.

4. The method for manufacturing a packaged medical device according to claim 3, wherein the partition portion is formed by opposite inner surfaces of the packaging bag being subjected to thermal fusion bonding.

5. The method for manufacturing a packaged medical device according to claim 4, wherein the partition portion is formed in such a manner that a fusion bonding strength thereof is lower than that of a peripheral portion of the packaging bag.

6. The method for manufacturing a packaged medical device according to any one of claims 3 to 5, wherein, in the communication step, at least a part of the partition portion is broken by pressing the second sub-chamber to cause the first sub-chamber and the second sub-chamber to communicate with each other.

7. The method for manufacturing a packaged medical device according to any one of claims 1 to 6, wherein radiation used for irradiation in the irradiation step is an electron beam.

8. The method for manufacturing a packaged medical device according to any one of claims 2 to 7, wherein the medical device is a catheter having a drug eluting stent or a drug eluting balloon.
